# FASCICULE DE BREVET EUROPEEN

(11) **EP 3 562 450 B1**
(45) Date de publication et mention de la délivrance du brevet: **27.01.2021**
(21) Numéro de dépôt: 17832791.2
(22) Date de dépôt: 28.12.2017
(51) Int. Cl.: A61F 9/013, A61M 37/00, A61F 9/007

(54) **INSTRUMENT A MAIN CHIRURGICAL**
CHIRURGISCHES HANDINSTRUMENT
SURGICAL HAND INSTRUMENT

(30) Priorité: 02.01.2017 FR 1750002
(43) Date de publication de la demande: 06.11.2019
(73) Titulaire: Neoris, 75004 Paris (FR)
(72) Inventeur: FERRARI, Francesco, 67100 Strasbourg (FR)
(74) Mandataire: Cabinet Bleger-Rhein-Poupon
(86) Numéro de dépôt international: PCT/FR2017/053858
(87) Numéro de publication internationale: WO 2018/122537

(56) Documents cités:
- WO-A1-00/35392
- WO-A1-96/06584
- US-A- 5 810 862
- US-A1- 2005 203 554
- US-A1- 2014 107 631

## Description

La présente invention a pour objet un instrument à main chirurgical utilisé dans le domaine de la chirurgie de l'œil, et plus particulièrement, pour une opération de kératopigmentation, telle que décrite dans le document US2014/0107631.

L'opération de kératopigmentation telle que décrite dans le document US2014/0107631, consiste à créer dans la cornée une cavité annulaire coaxiale à la pupille, et à injecter un colorant dans cette cavité.

La principale difficulté de cette intervention réside dans la réalisation de la cavité annulaire et plus particulièrement dans la précision avec laquelle doit être faite la découpe au niveau des bords périphériques internes et externes. En effet, cette cavité est destinée à recevoir un colorant, et un bord inégal est susceptible de générer des coulures inesthétiques.

Selon le document US2014/0107631, on utilise d'une part un laser femtoseconde pour créer une cavité annulaire centrée sur l'axe visuel. Le laser ne pouvant pas couper près du limbe, c'est-à-dire à la frontière entre cornée et conjonctive, il est nécessaire de régulariser la forme et les dimensions de la cavité, notamment au niveau de son bord externe, manuellement avec un instrument chirurgical.

Cet instrument à main chirurgical comporte un manche à une extrémité duquel est disposée une lame plate conformée en arc de cercle et qui s'étend selon un plan faisant un angle avec l'axe principal dudit manche pour faciliter son manœuvrement. La lame qui est d'une largeur adaptée à celle que l'on veut donner à la cavité annulaire, est destinée à être engagée dans cette dernière après avoir pratiqué une entaille radiale d'introduction, en sorte de pouvoir conformer cette cavité et en façonner les bords. Cet instrument à main chirurgical est décliné en deux versions, l'une où la lame en arc de cercle tourne à partir du manche dans un sens et une autre où la lame tourne dans l'autre sens, en sorte de pouvoir traiter la cavité de part et d'autre de l'entaille radiale.

Un tel instrument, dans sa configuration actuellement connue, permet d'affiner la réalisation de la cavité annulaire au niveau de ses contours, sans toutefois atteindre un résultat optimal, notamment au niveau du bord externe de la cavité, à proximité du limbe.

La présente invention a pour but de résoudre ce problème, en proposant une version améliorée de l'instrument à main chirurgical précité.

L'instrument à main chirurgical selon l'invention, utilisé dans le domaine de la chirurgie de l'œil, et plus particulièrement pour une opération de kératopigmentation, comporte un manche à une extrémité duquel est disposée une lame plate conformée en arc de cercle et qui s'étend selon un plan faisant un angle avec l'axe principal dudit manche, et il se caractérise en ce que l'extrémité distale de ladite lame présente une forme arrondie, est d'une largeur supérieure à celle du corps de ladite lame, et présente un bord tranchant.

Selon une caractéristique additionnelle de l'instrument à main chirurgical selon l'invention, le tranchant se présente sous la forme d'un biseau réalisé sur une seule face de la lame, celle du côté du manche.

Selon une autre caractéristique additionnelle de l'instrument à main chirurgical selon l'invention, le bord tranchant est localisé au niveau du bord extrême de la partie distale, et se prolonge sur une partie du bord périphérique externe.

Selon une autre caractéristique additionnelle de l'instrument à main chirurgical selon l'invention, l'extrémité distale de la lame se présente sous la forme d'une portion d'un disque, dont le diamètre supérieur à la largeur du corps de la lame.

Selon une autre caractéristique additionnelle de l'instrument à main chirurgical selon l'invention, l'extrémité distale de la lame qui se présente sous la forme d'une portion d'un disque est tangente au bord interne de la lame.

Selon une autre caractéristique additionnelle de l'instrument à main chirurgical selon l'invention, la largeur de l'extrémité distale de la lame est supérieure à celle du corps de la lame selon un rapport de 1 à 1,2.

Selon une autre caractéristique additionnelle de l'instrument à main chirurgical selon l'invention, la lame présente une forme d'un arc de cercle qui s'étend sur un secteur angulaire d'angle de 120°.

Selon une autre caractéristique additionnelle de l'instrument à main chirurgical selon l'invention, l'angle que fait le plan dans lequel s'étend la lame avec l'axe principal de l'instrument, est d'une valeur de 55°.

Selon une autre caractéristique additionnelle de l'instrument à main chirurgical selon l'invention, la lame est portée par des moyens réversibles de solidarisation au manche.

Selon une autre caractéristique additionnelle de l'instrument à main chirurgical selon l'invention, au moins la lame est réalisée en une matière plastique, ou en un matériau composite.

Le matériau composite peut consister en un polymère haute performance, éventuellement chargé de fibres de renfort.

De manière avantageuse, une lame amovible peut être à usage unique, qu'elle soit réalisée en métal, en une matière plastique ou en un matériau composite.

Il est également possible que l'instrument selon l'invention soit intégralement à usage unique, il est alors de préférence réalisé en une matière plastique ou en un matériau composite.

Les avantages et les caractéristiques de l'instrument à main chirurgical selon l'invention, ressortiront plus clairement de la description qui suit et qui se rapporte au dessin annexé, lequel en représente plusieurs modes de réalisation non limitatifs.

Dans le dessin annexé :
- la figure 1 représente une vue en plan d'un instrument à main chirurgical selon l'invention.
- la figure 2 représente une vue en plan sous un autre angle du même instrument à main chirurgical.
- la figure 3 représente une vue du même instrument à main chirurgical selon la flèche A de la figure 2.

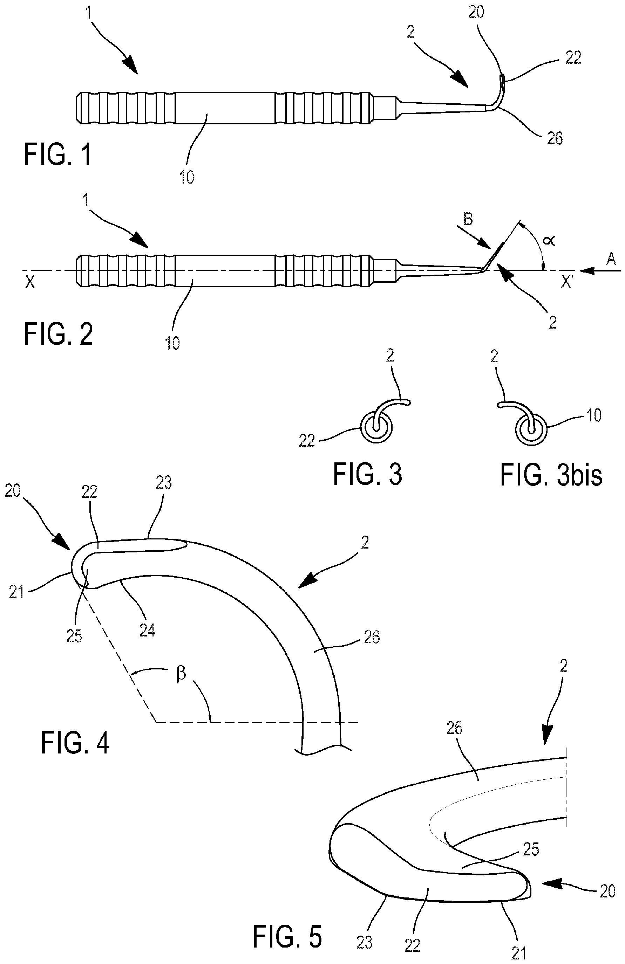

- la figure 3bis représente la même vue d'un instrument à main chirurgical symétrique.
- la figure 4 représente une vue sous un autre angle de la même partie de l'instrument, selon la flèche B de la figure 2.
- la figure 5 représente une vue en perspective de la même partie de l'instrument.

En référence aux figures 1 et 2, on peut voir un instrument à main chirurgical 1 selon l'invention. Il comporte un manche 10 destiné à être tenu à la main et à en permettre la manipulation, et à une extrémité une lame 2.

Comme cela est visible sur la figure 2, la lame 2 s'étend dans un plan faisant un angle α avec l'axe principal XX' de l'instrument 1, d'une valeur en l'occurrence de, non limitativement, 55°. Cette valeur est bien entendu modifiable en fonction des habitudes du praticien.

Comme cela est plus particulièrement visible sur la figure 4, la lame 2 présente une forme d'arc de cercle qui s'étend selon sur un secteur angulaire d'angle β de, non limitativement, 120°, ce qui permet au praticien d'aller suffisamment loin dans son geste toit en en gardant la maîtrise.

En référence à la figure 3 on peut voir que la lame 2 de l'instrument 1 tourne dans un sens, tandis que sur la figure 3bis la lame 2 d'un autre instrument 1 tourne dans l'autre sens.

Sur la figure 4, on peut voir que la lame 2 comporte une largeur constante, tandis que son extrémité distale 20 présente un élargissement 25 et affecte une forme arrondie. D'autre part, le bord extrême 21 de la partie distale 20 comporte, supérieurement, c'est-à-dire du côté du manche 10, également visible sur la figure 5, un biseau 22 qui se prolonge en partie sur le bord périphérique externe 23 de la lame 2, en sorte de former un bord tranchant, tandis que le bord périphérique interne 24 n'est pas tranchant.

De manière préférentielle, l'élargissement 25 se présente sous la forme d'une portion de disque dont le diamètre est supérieur à la largeur du corps 26 de la lame 2, en l'occurrence le diamètre est 20% plus grand.

On notera que cet élargissement 25 en forme de portion de disque est tangent au bord périphérique interne 24, en sorte que cet élargissement 25 se traduit par un débordement du côté extérieur, soit du côté du bord périphérique externe 23.

L'élargissement 25 de la partie distale 20, et sa localisation en partie extrême et sur le bord périphérique externe, permet de disséquer la cornée en direction de la conjonctive avec un plus grand contrôle, et donc avec une plus grande précision. De même, la localisation de la partie tranchante autorise une meilleure gestion de l'opération de coupe.

L'instrument 1 à main chirurgical selon l'invention, permet au praticien de réaliser un geste plus précis, lequel autorise une découpe optimale et donc un résultat supérieur du point de vue esthétique.

L'instrument 1 peut être réalisé dans un métal apte à supporter des opérations de désinfection et de stérilisation.

Il peut également être prévu à usage unique, entièrement ou partiellement. Dans ce cas il peut présenter des caractéristiques spécifiques.

Ainsi, la lame 2 peut être prévue amovible au travers de moyens réversibles de solidarisation au manche 10, lesquels peuvent consister, non limitativement, en des moyens d'encliquetage ou de vissage.

Il est également possible que la lame 2, qu'elle soit amovible ou non, ou bien tout ou partie de l'instrument 1, soient réalisés, non limitativement, dans un matériau tel qu'une matière plastique ou un matériau composite, thermoplastique ou thermodurcissable, de type polymère haute performance, éventuellement chargé de fibres de renfort.

## Revendications

1. Instrument à main chirurgical (1) utilisé dans le domaine de la chirurgie de l'œil, et plus particulièrement pour une opération de kératopigmentation, comportant un manche (10) à une extrémité duquel est disposée une lame (2) plate conformée en arc de cercle et qui s'étend selon un plan faisant un angle (a) avec l'axe principal (XX') dudit manche (10), **caractérisé en ce que** l'extrémité distale (20) de ladite lame (2) présente une forme arrondie, est d'une largeur supérieure à celle du corps (26) de ladite lame (2), et présente un bord tranchant (22).

2. Instrument à main chirurgical (1) selon la revendication 1 ou la revendication 2, **caractérisé en ce que** le tranchant se présente sous la forme d'un biseau (22) réalisé sur une seule face de la lame (2), celle du côté du manche (10).

3. Instrument à main chirurgical (1) selon la revendication 1 ou la revendication 2, **caractérisé en ce que** le bord tranchant (22) est localisé au niveau du bord extrême (21) de la partie distale (20), et se prolonge sur une partie du bord périphérique externe (23).

4. Instrument à main chirurgical (1) selon l'une quelconque des revendications 1 à 3, **caractérisé en ce que** l'extrémité distale (20) de la lame (2) se présente sous la forme d'une portion d'un disque (25), dont le diamètre supérieur à la largeur du corps (26) de la lame (2).

5. Instrument à main chirurgical (1) selon la revendication 1 ou la revendication 4, **caractérisé en ce que** l'extrémité distale (20) de la lame (2) qui se présente sous la forme d'une portion d'un disque est tangente au bord interne (24) de la lame (2).

6. Instrument à main chirurgical (1) selon l'une quelconque des revendications 1 à 5, **caractérisé en ce que** la largeur de l'extrémité distale (20) de la lame (2) est supérieure à celle du corps (26) de la lame (2) selon un rapport de 1 à 1,2.

7. Instrument à main chirurgical (1) selon l'une quelconque des revendications 1 à 6, **caractérisé en ce que** la lame (2) présente une forme d'un arc de cercle qui s'étend sur un secteur angulaire d'angle (β) de 120°.

8. Instrument à main chirurgical (1) selon l'une quelconque des revendications 1 à 7, **caractérisé en ce que** l'angle (a) que fait le plan dans lequel s'étend la lame (2) avec l'axe principal (XX') de l'instrument, est d'une valeur de 55°.

9. Instrument à main chirurgical (1) selon l'une quelconque des revendications 1 à 8, **caractérisé en ce que** la lame (2) est portée par des moyens réversibles de solidarisation au manche (10).

10. Instrument à main chirurgical (1) selon l'une quelconque des revendications 1 à 9, **caractérisé en ce qu'**au moins la lame (2) est réalisée en une matière plastique ou en un matériau composite.

## Patentansprüche

1. Chirurgisches Handinstrument (1), das auf dem Gebiet der Augenchirurgie und insbesondere für eine Keratopigmentierungsoperation verwendet wird und einen Griff (10) umfasst, an dessen einem Ende eine kreisbogenförmige flache Klinge (2) angeordnet ist, der sich entlang einer Ebene erstreckt, die einen Winkel (a) mit der Hauptachse (XX') des besagten Griffs (10) einschließt, **dadurch gekennzeichnet, dass** das distale Ende (20) der Klinge (2) eine abgerundete Form aufweist, eine Breite hat, die größer als diejenige des Körpers (26) der Klinge (2) ist, und eine Schneidkante (22) aufweist.

2. Chirurgisches Handinstrument (1) nach Anspruch 1 oder Anspruch 2, **dadurch gekennzeichnet, dass** die Schneide die Form einer Abschrägung (22) aufweist, die auf einer Fläche der Klinge (2), derjenigen an der Seite des Griffs (10), hergestellt ist.

3. Chirurgisches Handinstrument (1) nach Anspruch 1 oder Anspruch 2, **dadurch gekennzeichnet, dass** sich die Schneidkante (22) im Bereich der äußersten Kante (21) des distalen Teils (20) befindet und sich über einen Teil der äußeren Umfangskante (23) erstreckt.

4. Chirurgisches Handinstrument (1) nach irgendeinem der Ansprüche 1 bis 3, **dadurch gekennzeichnet, dass** das distale Ende (20) der Klinge (2) in Form eines Teils einer Scheibe (25) vorliegt, dessen Durchmesser größer ist als die Breite des Körpers (26) der Klinge (2).

5. Chirurgisches Handinstrument (1) nach Anspruch 1 oder Anspruch 4, **dadurch gekennzeichnet, dass** das distale Ende (20) der Klinge (2), das in Form eines Teils einer Scheibe vorliegt, tangential zu der Innenkante (24) der Klinge (2) ist.

6. Chirurgisches Handinstrument (1) nach irgendeinem der Ansprüche 1 bis 5, **dadurch gekennzeichnet, dass** die Breite des distalen Endes (20) der Klinge (2) größer ist als diejenige des Körpers (26) der Klinge (2) in einem Verhältnis von 1 zu 1,2.

7. Chirurgisches Handinstrument (1) nach irgendeinem der Ansprüche 1 bis 6, **dadurch gekennzeichnet, dass** die Klinge (2) die Form eines Kreisbogens hat, der sich über einen Winkelabschnitt mit einem Winkel (β) von 120° erstreckt.

8. Chirurgisches Handinstrument (1) nach irgendeinem der Ansprüche 1 bis 7, **dadurch gekennzeichnet, dass** der Winkel (a), den die Ebene, in der sich die Klinge (2) erstreckt, mit der Hauptachse (XX') des Instruments einschließt, einen Wert von 55° hat.

9. Chirurgisches Handinstrument (1) nach irgendeinem der Ansprüche 1 bis 8, **dadurch gekennzeichnet, dass** die Klinge (2) durch reversible Mittel zum festen Verbinden mit dem Griff (10) getragen wird.

10. Chirurgisches Handinstrument (1) nach irgendeinem der Ansprüche 1 bis 9, **dadurch gekennzeichnet, dass** mindestens die Klinge (2) aus einem Kunststoffmaterial oder einem Verbundmaterial besteht.

## Claims

1. A surgical hand instrument (1) used in the field of the eye surgery, and more particularly for a keratopigmentation operation, including a handle (10) at one end of which is arranged a flat blade (2) shaped in the manner of a circular arc and which extends along a plane forming an angle (α) with the main axis (XX') of said handle (10), wherein the distal end (20) of said blade (2) has a rounded shape, and has a width that is larger than that of the body (26) of said blade (2), and has a cutting edge (22).

2. The surgical hand instrument (1) according to claim 1 or claim 2, wherein the cutting edge is in the form of a bevel (22) made on one surface of the blade (2), the one on the side of the handle (10).

3. The surgical hand instrument (1) according to claim 1 or claim 2, wherein the cutting edge (22) is located at the level of the outermost edge (21) of the distal portion (20), and extends over a portion of the outer peripheral edge (23).

4. The surgical hand instrument (1) according to any one of claims 1 to 3, wherein the distal end (20) of the blade (2) is in the form of a portion of a disc (25) the diameter of which is larger than the width of the body (26) of the blade (2).

5. The surgical hand instrument (1) according to claim 1 or claim 4, wherein the distal end (20) of the blade (2), which is in the form of a portion of a disc, is tangent to the internal edge (24) of the blade (2).

6. The surgical hand instrument (1) according to any one of claims 1 to 5, wherein the width of the distal end (20) of the blade (2) is larger than that of the body (26) of the blade (2) in a ratio of 1 to 1.2.

7. The surgical hand instrument (1) according to any one of claims 1 to 6, wherein the blade (2) has the shape of a circular arc, which extends over an angular section having an angle (β) of 120°.

8. The surgical hand instrument (1) according to any one of claims 1 to 7, wherein the angle (α), which is formed by the plane in which the blade (2) extends with the main axis (XX') of the instrument, has a value of 55°.

9. The surgical hand instrument (1) according to any one of claims 1 to 8, wherein the blade (2) is carried by reversible means for making it integral with the handle (10).

10. The surgical hand instrument (1) according to any one of claims 1 to 9, wherein at least the blade (2) is made out of a plastic material or a composite material.
